# EUROPEAN PATENT APPLICATION

(11) **EP 3 872 072 A1**
(43) Date of publication of application: **01.09.2021**
(21) Application number: 19875274.3
(22) Date of filing: 21.10.2019
(51) Int. Cl.: C07D 401/12, A61K 31/4709, A61P 35/00

(54) **CRYSTAL FORM OF MALEATE OF TYROSINE KINASE INHIBITOR AND PREPARATION METHOD THEREFOR**

(30) Priority: 22.10.2018 CN 201811231321
(71) Applicant: Jiangsu Hengrui Medicine Co., Ltd., Lianyungang, Jiangsu 222047 (CN)
(72) Inventor: QIU, Zhenjun, Lianyungang, Jiangsu 222047 (CN); ZHANG, Quanliang, Lianyungang, Jiangsu 222047 (CN); WEI, Yanli, Lianyungang, Jiangsu 222047 (CN); CAO, Yongxing, Lianyungang, Jiangsu 222047 (CN); YANG, Junran, Lianyungang, Jiangsu 222047 (CN); MA, Yahui, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2019/112216
(87) International publication number: WO 2020/083188

(57) **Abstract**

Provided are a crystal form of a maleate of a tyrosine kinase inhibitor and a preparation method therefor. Specifically, provided are I crystal form, a II crystal form, a III crystal form, a IV crystal form and a V crystal form of the compound as shown in formula (I) and a preparation method therefor. The new crystal form has a good stability, thereby making same better to use in clinical treatments.

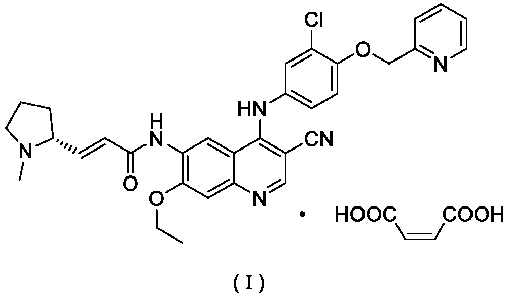

## Description

The present application claims the priority of Chinese patent application CN201811231321.7 filed on October 22,2018. The contents of the Chinese patent application are incorporated herein by reference in their entireties.

### Technical Field

The present disclosure relates to a crystal form of maleate of tyrosine kinase inhibitor and preparation method therefor.

### Prior Art

Studies have shown that more than 50% of proto-oncogenes and oncogene products have tyrosine kinase activity, and their abnormal expression will lead to tumorigenesis. Tyrosine kinase inhibitors have been on the market since 2001 and have become a new class of anticancer drugs that have emerged.

Epidermal growth factor receptor (EGFR) is a member of the receptor tyrosine kinase family, the epidermal growth factor receptor pathway plays a very important role in the occurrence and development of tumors, and it has become the one of the most important targets for researching and development in the field of tumor treatment. Such drugs that have been on the market include erlotinib, gefitinib and lapatinb (Tykerb, GW572016).

WO2011029265A1 has disclosed an epidermal growth factor receptor (EGFR) inhibitor, whose chemical name is (*R*, *E*)-*N*-(4-(3-chloro-4-(pyridin-2-ylmethoxy)phenylamino)-3-cyano-7-ethoxyquinolin-6-yl)-3-(1-methylpyrrolidin-2-yl)acrylamide, the drug molecule has obvious pharmacokinetic and pharmacodynamic advantages, the structure is as shown in formula (II):

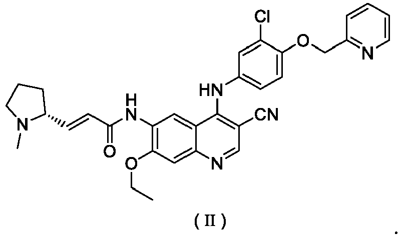

CN102675287A has disclosed a monomaleate of the compound represented by formula (II), and the structure of the salt is as shown in formula (I):

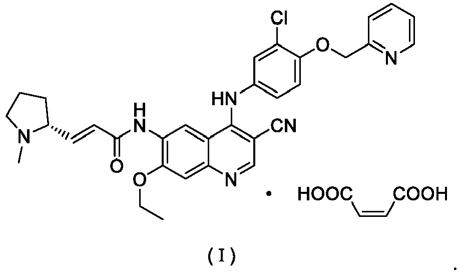

CN102675287A has also disclosed a dimaleate form of the compound represented by formula (II), and the biological activity test results show that the dimaleate of the compound represented by formula (II) has high activity. CN103974949A has disclosed a crystal form of the dimaleate of the compound represented by formula (II). The compound represented by formula (II) is currently being developed in the form of dimaleate.

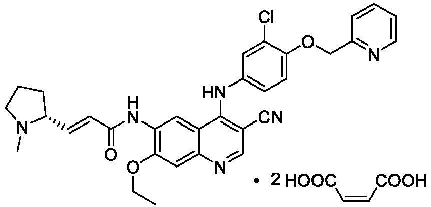

The crystal structure of the pharmaceutical active ingredient often affects the chemical and physical stability of the drug, the difference in crystallization conditions and storage conditions may lead to changes in the crystal structure of the compound, sometimes accompanied by the production of other crystalline forms. Therefore, it is necessary to improve the properties of said products, we need in-depth research to find new crystal forms with high crystal purity and good chemical stability.

### Content of the present invention

The purpose of the present disclosure is to provide a novel crystal form of the compound represented by formula (I), which has good stability and can be better applied in clinics.

The present disclosure in one aspect provides a I crystal form of a compound represented by formula (I), which has an X-ray powder diffraction pattern spectrum comprising characteristic peaks at 2θ angles of 6.57, 8.12, 9.76, 10.77, 14.98, 15.89, 20.97, 21.64, 22.06 and 22.61.

In some embodiments, the present disclosure provides a I crystal form of a compound represented by formula (I), which has an X-ray powder diffraction pattern spectrum comprising characteristic peaks at 2θ angles of 6.57, 8.12, 9.76, 10.77, 14.47, 14.98, 15.28, 15.89, 20.97, 21.64, 22.06, 22.61, 24.00, 25.62 and 26.46.

In some embodiments, the present disclosure provides a I crystal form of a compound represented by formula (I), which has an X-ray powder diffraction pattern spectrum comprising characteristic peaks at 2θ angles of 6.57, 8.12, 9.76, 10.77, 12.42, 13.11, 14.47, 14.98, 15.28, 15.89, 16.29, 16.49, 17.13, 17.46, 18.92, 19.56, 19.83, 20.29, 20.97, 21.64, 22.06, 22.61, 22.99 , 24.00, 24.60, 25.62, 26.46, 27.30, 27.99, 29.05, 30.19, 30.69, 31.90, 33.88 and 36.07.

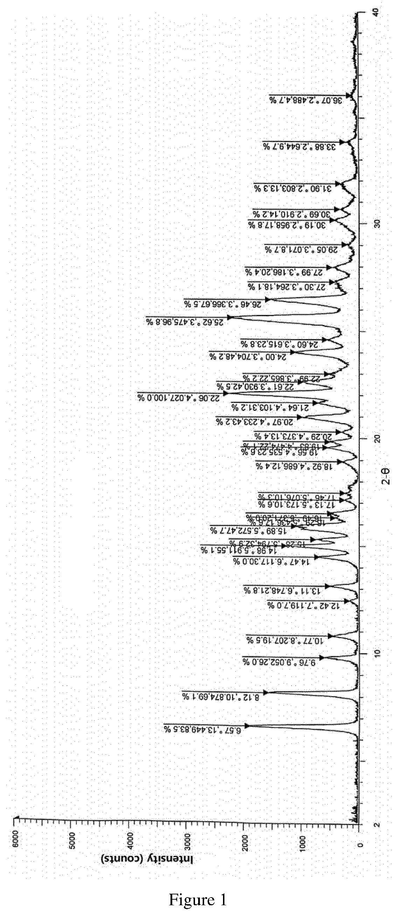

In some embodiments, the present disclosure provides a I crystal form of the compound represented by formula (I), which has an X-ray powder diffraction pattern spectrum as shown in Figure 1.

The present disclosure further provides a method for preparing the I crystal form of the compound represented by formula (I), the method comprising:

mixing and slurring the compound represented by formula (I) with a solvent and filtering the crystals obtained, the solvent is selected from one or more of water and tetrahydrofuran; or mixing the compound represented by formula (I) with a solvent to volatilize and crystallize, and the solvent is one or more selected from ethanol, isopropanol, n-propanol, acetone, acetonitrile, 2-butanone, dimethyl sulfoxide, nitromethane (MN), propylene glycol methyl ether (PM), isoamylol (IAA) and acetophenone (ACP); or mixing the compound represented by formula (II) with maleic acid and a solvent to precipitate a solid and filtering the crystals obtained, the solvent is one or more selected from isopropanol, water, and dichloromethane, preferably a mixed solvent of isopropanol/water or dichloromethane.

The present disclosure in one aspect provides a II crystal form of a compound represented by formula (I), which has an X-ray powder diffraction pattern spectrum comprising characteristic peaks at 2θ angles of 6.340, 9.030, 10.232, 11.503, 18.282, 19.399, 20.865 and 21.558.

In some embodiments, the present disclosure has provided a II crystal form of a compound represented by formula (I), which has an X-ray powder diffraction pattern spectrum comprising characteristic peaks at 2θ angles of 6.340, 9.030, 10.232, 11.503, 12.629, 13.637, 14.526, 16.170, 17.639, 18.282, 19.399, 20.865, 21.558, 22.078, 22.616, 23.562, 24.479, 25.801, 27.601, 28.139, 29.671, 31.893 and 33.887.

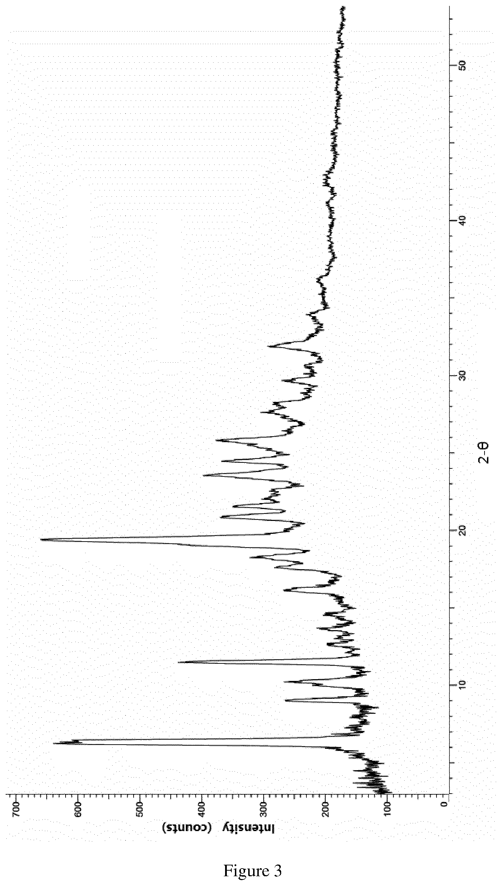

In some embodiments, the present disclosure has provided a II crystal form of a compound represented by formula (I), which has an X-ray powder diffraction pattern spectrum as shown in Figure 3.

The present disclosure further provides a method for preparing the II crystal form of the compound represented by formula (I), wherein the method comprising: mixing and slurring a crystal form of the compound of formula (I) with tetrahydrofuran to precipitate a solid, and filtering the crystals obtained. Preferably, the crystal form of the compound represented by formula (I) is I crystal form.

The present disclosure in one aspect provides a III crystal form of a compound represented by formula (I), which has an X-ray powder diffraction pattern spectrum comprising characteristic peaks at 2θ angles of 6.291, 6.547, 8.561, 9.908, 10.401, 17.381, 19.326 and 23.741.

In some embodiments, the present disclosure has provided a III crystal form of a compound represented by formula (I), which has an X-ray powder diffraction pattern spectrum comprising characteristic peaks at 2θ angles of 4.864, 5.516, 6.291, 6.547, 8.068, 8.561, 9.908, 10.401, 11.603, 13.267, 13.819, 14.725, 16.270, 17.381, 18.398, 19.326, 20.125, 21.040, 21.498, 22.250, 23.741, 24.426, 25.795, 26.765, 28.530 and 31.815.

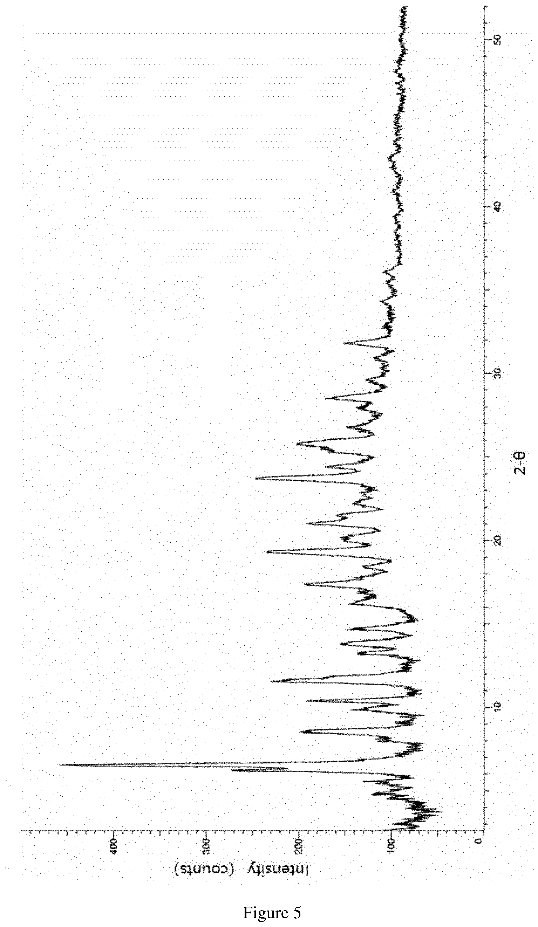

In some embodiments, the present disclosure has provided a III crystal form of a compound represented by formula (I), which has an X-ray powder diffraction pattern spectrum as shown in Figure 5.

The present disclosure further provides a method for preparing the crystal form III of the compound represented by formula (I), wherein the method comprising: mixing the compound represented by formula (II) with maleic acid and acetone to precipitate a solid, and filtering the crystals obtained.

The present disclosure in one aspect provides a IV crystal form of a compound represented by formula (I), which has an X-ray powder diffraction pattern spectrum comprising characteristic peaks at 2θ angles of 5.638, 9.417, 11.054, 12.386, 15.218, 15.639, 17.074 and 18.369.

In some embodiments, the present disclosure has provided a IV crystal form of a compound represented by formula (I), which has an X-ray powder diffraction pattern spectrum comprising characteristic peaks at 2θ angles of 5.638, 9.417, 11.054, 12.386, 15.218, 15.639, 17.074, 18.369, 22.779, 23.414, 25.384, 26.426 and 28.685.

In some embodiments, the present disclosure has provided a IV crystal form of a compound represented by formula (I), which has an X-ray powder diffraction pattern spectrum comprising characteristic peaks at 2θ angles of 5.638, 8.268, 8.772, 9.417, 11.054, 12.386, 13.739, 15.218, 15.639, 16.312, 17.074, 18.369, 19.152, 20.439, 21.907, 22.307, 22.779, 23.414, 24.146, 24.837, 25.384, 25.852, 26.426, 26.774, 28.685, 29.782, 31.620 and 32.482.

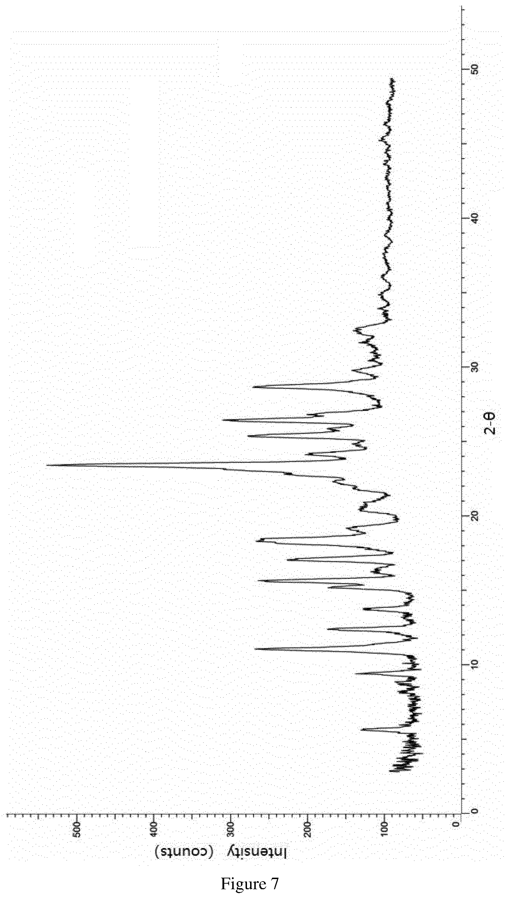

In some embodiments, the present disclosure has provided a IV crystal form of a compound represented by formula (I), which has an X-ray powder diffraction pattern spectrum as shown in Figure 7.

The present disclosure further provides a method for preparing the IV crystal form of the compound represented by formula (I), the method comprising:

mixing the compound represented by formula (II) with maleic acid and a solvent to precipitate a solid, and filtering the crystals obtained, the solvent can be one or more selected from n-propanol, isopropyl acetate, 2-butanone, isopropanol, and ethanol, preferably ethanol.

The present disclosure in one aspect provides a V crystal form of a compound represented by formula (I), which has an X-ray powder diffraction pattern spectrum comprising characteristic peaks at 2θ angles of 5.469, 5.477, 6.512, 10.376, 11.593, 18.241, 19.386, 21.028 and 22.286.

In some embodiments, the present disclosure has provided a V crystal form of a compound represented by formula (I), which has an X-ray powder diffraction pattern spectrum comprising characteristic peaks at 2θ angles of 5.469, 5.477, 6.512, 10.376, 11.593, 13.220, 14.708, 15.600,16.492, 18.241, 19.386, 21.028, 22.286, 22.747, 23.758, 24.693, 25.509, 25.926, 26.563, 27.837, 29.792, 30.727 and 32.086.

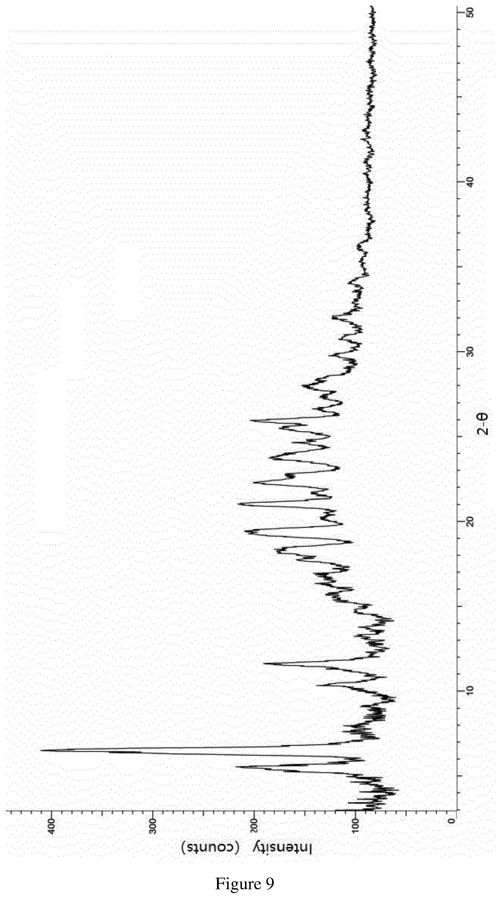

In some embodiments, the present disclosure has provided a V crystal form of the compound represented by formula (I), which has an X-ray powder diffraction pattern spectrum as shown in Figure 9.

The present disclosure further provides a method for preparing the V crystal form of the compound represented by formula (I), the method comprising: mixing the compound represented by formula (II) with maleic acid and a solvent to precipitate a solid, filtering the crystals obtained, the solvent can be 1,4-dioxane and/or tetrahydrofuran,

The present disclosure further relates to a pharmaceutical composition comprising one or more of the I crystal form, II crystal form, III crystal form, IV crystal form and V crystal form of the compound represented by formula (I) and one or more pharmaceutically acceptable carriers, diluents or excipients.

The present disclosure further relates to a pharmaceutical composition prepared by mixing one or more of the I crystal form, II crystal form, III crystal form, IV crystal form and V crystal form of the compound represented by formula (I) with one or more pharmaceutically acceptable carriers, diluents or excipients.

The present disclosure further relates to a method for preparing the pharmaceutical composition comprising the compound represented by formula (I) or the pharmaceutically acceptable salt thereof, wherein the method comprises mixing one or more of the I crystal form, the II crystal form, III crystal form, IV crystal form, and V crystal form of the compound represented by formula (I) with one or more pharmaceutically acceptable carriers, diluents or excipients.

The pharmaceutical composition of the present disclosure can be made into any pharmaceutically acceptable dosage form. For example, the crystal forms or pharmaceutical preparations of the present disclosure can be formulated as tablets, capsules, pills, granules, solutions, suspensions, syrups, injections (including injections, sterile powders for injections, and concentrated solutions for injections), suppositories, inhalants or sprays.

The present disclosure further relates to I crystal form, II crystal form, III crystal form, IV crystal form, V crystal form of the compound represented by formula (I), or the pharmaceutical composition described in the present disclosure for use in manufacturing a medicament for the treatment and/or prevention of a disease or a condition related to protein kinase, wherein the protein kinase is selected from EGFR receptor tyrosine kinase or HER-2 receptor tyrosine kinase, the disease or condition is preferably cancer, and the cancer is preferably lung cancer, breast cancer, epidermal squamous cell carcinoma or gastric cancer.

Through X-ray powder diffraction pattern (XRPD) spectrum and differential scanning calorimetry (DSC) analysis, the crystal form obtained in the present disclosure is subjected to structure determination and crystal form study.

The crystallization method of the crystal form in the present disclosure is conventional, such as volatilization crystallization, cooling crystallization, or crystallization at room temperature.

The starting material used in the method for preparing the crystal form of the present disclosure can be any form of the compound represented by formula (I), or the reaction of the compound represented by formula (II) with maleic acid, or the dimaleate salt of the compound represented by formula (II) with one maleic acid removed, and specific forms include but are not limited to: amorphous, arbitrary crystal, hydrate, solvate, and the like.

In the description and claims of this application, unless otherwise specified, the scientific and technical terms used herein have the meanings commonly understood by those skilled in the art. However, in order to better understand the present disclosure, some of the definitions and explanations of related terms are provided below. In addition, when the definitions and explanations of the terms provided in this application are inconsistent with the meanings commonly understood by those skilled in the art, the definitions and explanations of the terms provided in this application shall prevail.

The "slurring" in the present disclosure refers to a method of purification using the characteristics of poor solubility of substances in solvents, but good solubility of impurities in solvents, the slurring purification can decolorize, change the crystal form or remove a small amount of impurities.

The "X-ray powder diffraction pattern spectrum or XRPD" in this disclosure refers to according to Bragg formula 2d sin θ = nλ (where λ is the wavelength of the X-ray, λ=1.5406Å, and the diffraction order n is any positive integer, generally the first-order diffraction peak is taken, n=1), when the X-ray is incident on the atomic plane of the crystal or part of the crystal sample with a d lattice plane spacing at a grazing angle θ (the complementary angle of the incident angle, also called the Bragg angle), the Bragg equation can be satisfied, and this set of X-ray powder diffraction pattern spectrum can be measured.

The "X-ray powder diffraction pattern spectrum or XRPD" in the present disclosure is a pattern spectrum obtained by using Cu-Kα radiation in an X-ray powder diffractometer. As described about, λ=1.5406Å.

The "differential scanning calorimetry analysis or DSC" in the present disclosure refers to the measurement of the temperature difference and heat flow difference between the sample and the reference during the temperature rise or constant temperature, which is used to characterize all the physical and chemical changes related to the thermal effect and obtain the phase change information of the sample.

The "2θ or 2θ angle" in the present disclosure refers to the diffraction angle, θ is the Bragg angle, and the unit is ° or degree, and the error range of 2θ is ±0.3 or ±0.2 or ±0.1.

The "interplanar spacing or interplanar spacing (d value)" in the present disclosure refers to three non-parallel unit vectors a, b, and c selected from the spatial lattice connecting two adjacent lattice points, by which the matrix is divided into juxtaposed parallelepiped units, called interplanar spacing. The spatial lattice is divided according to the line of the determined parallelepiped units, and a set of straight-line grids are obtained, called the spatial lattices or lattices. Each of the spatial lattice and the lattice is used to reflect the periodicity of the crystal structure with geometric points and lines, for different crystal planes, the interplanar spacings (i.e., the distance between two adjacent parallel crystal planes) are different; the unit is Å or angstrom.

### The Beneficial Effects of the present invention

The crystal forms I and IV of the compound represented by formula (I) prepared by the present disclosure have high purity, and have good crystal stability under conditions of light, high temperature, and high humidity, small purity changes in HPLC, high chemical stability, and are more conducive to the effect of medicines. The solid properties of the II crystal form are poor, and the fluidity is poor. The reproducibility of the II, III, and V crystal forms is poor. The novel crystal form of the compound represented by the formula (I) obtained in the present disclosure can meet the medical requirements of production, transportation and storage, the production process of which is stable, repeatable and controllable, and suitable for industrial production.

### Brief Description of the drawings

Figure 1 is the XRPD pattern spectrum of the I crystal form of the compound represented by formula (I);
Figure 2 is a DSC pattern spectrum of the I crystal form of the compound represented by formula (I);
Figure 3 is the XRPD pattern spectrum of the II crystal form of the compound represented by formula (I);
Figure 4 is a DSC pattern spectrum of the II crystal form of the compound represented by formula (I);
Figure 5 is the XRPD pattern spectrum of the III crystal form of the compound represented by formula (I);
Figure 6 is a DSC pattern spectrum of the III crystal form of the compound represented by formula (I);
Figure 7 is the XRPD pattern spectrum of the IV crystal form of the compound represented by formula (I);
Figure 8 is a DSC pattern spectrum of the IV crystal form of the compound represented by formula (I);
Figure 9 is the XRPD pattern spectrum of the V crystal form of the compound represented by formula (I);
Figure 10 is a DSC pattern spectrum of the V crystal form of the compound represented by formula (I);
Figure 11 is the XRPD pattern spectrum of the amorphous compound represented by formula (I).

### Detailed Description of the Preferred Embodiment

Hereinafter, the present disclosure will be explained in more detail with the embodiments, the embodiments of the present disclosure are only used to illustrate the technical solutions of the present disclosure, and do not limit the essence and scope of the present disclosure.

### Experimental conditions of the equipment used in the test:

### 1. Differential Scanning Calorimeter (DSC)

Instrument model: Mettler Toledo DSC 1 STAR^{e} System
Purge gas: nitrogen
Heating rate: 10.0 °C/min
Temperature range:40-250 °C

### 2. Differential Scanning Calorimeter (DSC)

Instrument model: Mettler Toledo DSC 3+
Purge gas: nitrogen
Heating rate: 10.0 °C/min
Temperature range:25-300 °C

### 3. X-ray Powder Diffraction (XRPD)

Instrument model: BRUKER D8 DISCOVERY X-ray powder deiffractometer
Ray: monochrome Cu-Kα ray (λ=1.5406)
Scanning mode: θ/2θ, Scanning range: 5-48°
Voltage: 40KV, current: 40mA

### 4. X-ray Powder Diffraction (XRPD)

Instrument model: BRUKER D8 Focus X-ray powder deiffractometer
Ray: monochrome Cu-Kα ray (λ=1.5406)
Scanning mode: θ/2θ, Scanning range:2-40°
Voltage: 40KV, current: 40mA

Among them, the 2θ data with 2 decimal places is measured by BRUKER D8 Focus X-ray powder diffractometer.

### Embodiment 1

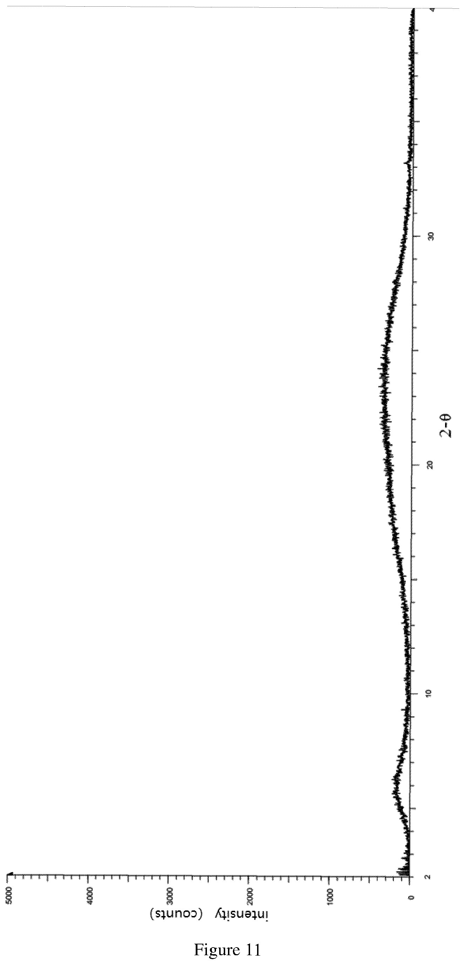

According to the method described in Embodiment 1 in CN102675287A, 0.5 g of the compound represented by formula (II) was added to obtain the amorphous form of the compound represented by formula (I), and its X-ray diffraction pattern spectrum is shown in Figure 11.

### Embodiment 2

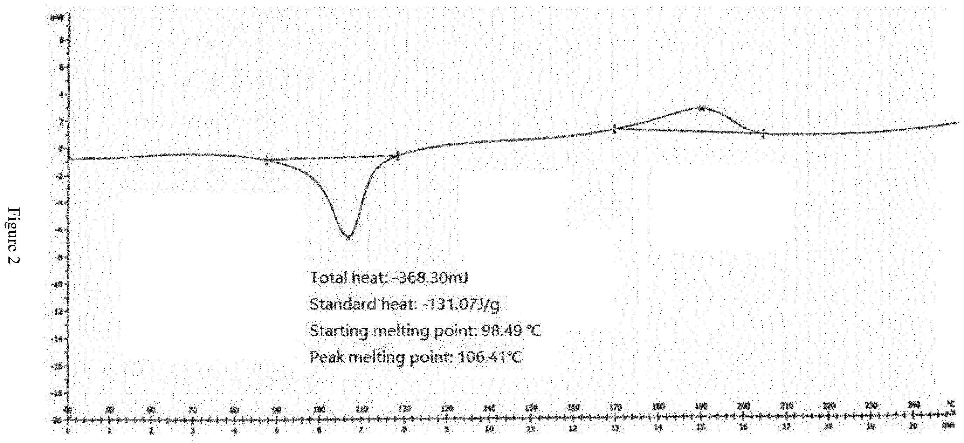

5.0g of the amorphous compound represented by formula (I) was placed in a reaction flask, 50mL of purified water was added thereto and the mixture was slurried and converted to crystals, then filtered with suction, the filter cake was washed with a small amount of purified water, and dried at 40°C to obtain the I crystal form of the compound represented by formula (I). Its X-ray diffraction pattern spectrum is shown in Figure 1, and its DSC pattern spectrum is shown in Figure 2, the characteristic peak positions are shown in the following table:

**Table 1: characteristic peak positions of the I crystal form**

| Number of the peaks | 2θ[°] | d[Å] | I[%] |
|---|---|---|---|
| Peak 1 | 6.57 | 13.449 | 83.5 |
| Peak 2 | 8.12 | 10.874 | 69.1 |
| Peak 3 | 9.76 | 9.052 | 26.0 |
| Peak 4 | 10.77 | 8.207 | 19.5 |
| Peak 5 | 12.42 | 7.119 | 7.0 |
| Peak 6 | 13.11 | 6.748 | 21.8 |
| Peak 7 | 14.47 | 6.117 | 30.0 |
| Peak 8 | 14.98 | 5.911 | 55.1 |
| Peak 9 | 15.28 | 5.794 | 32.9 |
| Peak 10 | 15.89 | 5.572 | 47.7 |
| Peak 11 | 16.29 | 5.436 | 17.6 |
| Peak 12 | 16.49 | 5.371 | 20.0 |
| Peak 13 | 17.13 | 5.173 | 10.6 |
| Peak 14 | 17.46 | 5.076 | 10.3 |
| Peak 15 | 18.92 | 4.686 | 12.4 |
| Peak 16 | 19.56 | 4.535 | 23.6 |
| Peak 17 | 19.83 | 4.474 | 22.1 |
| Peak 18 | 20.29 | 4.373 | 13.4 |
| Peak 19 | 20.97 | 4.233 | 43.2 |
| Peak 20 | 21.64 | 4.103 | 31.2 |
| Peak 21 | 22.06 | 4.027 | 100.0 |
| Peak 22 | 22.61 | 3.930 | 42.5 |
| Peak 23 | 22.99 | 3.865 | 22.2 |
| Peak 24 | 24.00 | 3.704 | 48.2 |
| Peak 25 | 24.60 | 3.615 | 23.8 |
| Peak 26 | 25.62 | 3.475 | 96.8 |
| Peak 27 | 26.46 | 3.366 | 67.5 |
| Peak 28 | 27.30 | 3.264 | 18.1 |
| Peak 29 | 27.99 | 3.186 | 20.4 |
| Peak 30 | 29.05 | 3.071 | 8.7 |
| Peak 31 | 30.19 | 2.958 | 17.8 |
| Peak 32 | 30.69 | 2.910 | 14.2 |
| Peak 33 | 31.90 | 2.803 | 13.3 |
| Peak 34 | 33.88 | 2.644 | 9.7 |
| Peak 35 | 36.07 | 2.488 | 4.7 |

### Embodiment 3

50 mg of the amorphous compound represented by formula (I) was placed in a reaction flask, 1 mL of tetrahydrofuran was added, the mixture was slurried and converted to crystals, then filtered with suction, and dried at 40°C to obtain the I crystal form of the compound represented by formula (I).

### Embodiment 4

Approximately 10 mg of the I crystal form of the compound represented by formula (I) was placed in a reaction flask, and 40 µL of ethanol was added for dissolution, the mixture was stirred at room temperature, and volatilized to crystallize to obtain the I crystal form of the compound represented by formula (I).

### Embodiment 5

Approximately 20 mg of the compound represented by formula (II) was weighed and 4 mg of maleic acid was added thereto, 90% IPA/H₂O 200µL was added at room temperature and stirred for dissolution, the stirring was continued and solids were precipitated, the mixture was then centrifuged, and the solid sample was dried in vacuum to obtain I crystal form of the compound represented by formula (I).

### Embodiment 6

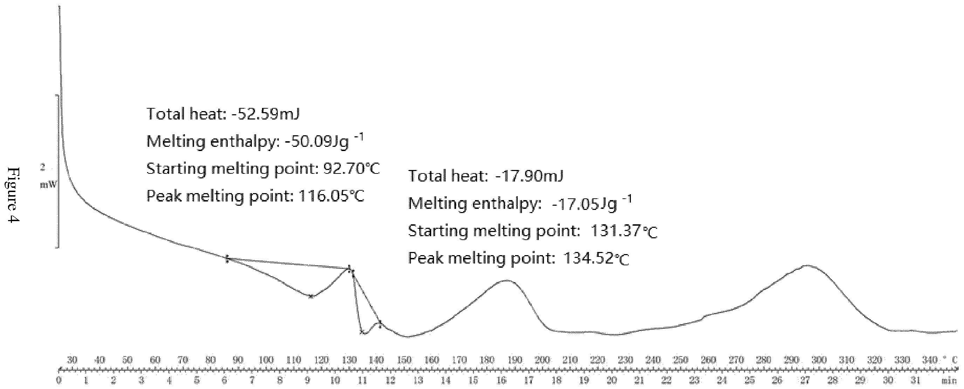

500 µL of tetrahydrofuran was added to about 10 mg of I crystal form of the compound represented by formula (I), the mixture was slurried at room temperature, centrifuged, and the solid part was dried in vacuum to obtain the II crystal form of the compound represented by formula (I). Its X-ray diffraction pattern spectrum is shown in Figure 3, the DSC pattern spectrum is shown in Figure 4, and the characteristic peak positions are shown in the table below:

**Table 2: characteristic peak positions of the II crystal form**

| Number of the peaks | 2θ[°] | d[Å] | I[%] |
|---|---|---|---|
| Peak 1 | 6.340 | 13.92901 | 100.0 |
| Peak 2 | 9.030 | 9.78560 | 28.1 |
| Peak 3 | 10.232 | 8.63810 | 26.2 |
| Peak 4 | 11.503 | 7.68685 | 58.5 |
| Peak 5 | 12.629 | 7.00355 | 7.1 |
| Peak 6 | 13.637 | 6.48824 | 8.2 |
| Peak 7 | 14.526 | 6.09317 | 9.4 |
| Peak 8 | 16.170 | 5.47718 | 18.4 |
| Peak 9 | 17.639 | 5.02414 | 16.1 |
| Peak 10 | 18.282 | 4.84890 | 21.0 |
| Peak 11 | 19.399 | 4.57199 | 89.3 |
| Peak 12 | 20.865 | 4.25396 | 25.0 |
| Peak 13 | 21.558 | 4.11881 | 20.9 |
| Peak 14 | 22.078 | 4.02300 | 9.9 |
| Peak 15 | 22.616 | 3.92835 | 7.2 |
| Peak 16 | 23.562 | 3.77274 | 28.7 |
| Peak 17 | 24.479 | 3.63346 | 23.0 |
| Peak 18 | 25.801 | 3.45026 | 24.9 |
| Peak 19 | 27.601 | 3.22924 | 9.3 |
| Peak 20 | 28.139 | 3.16862 | 9.2 |
| Peak 21 | 29.671 | 3.00846 | 8.1 |
| Peak 22 | 31.893 | 2.80374 | 13.7 |
| Peak 23 | 33.887 | 2.64321 | 2.8 |

### Embodiment 7

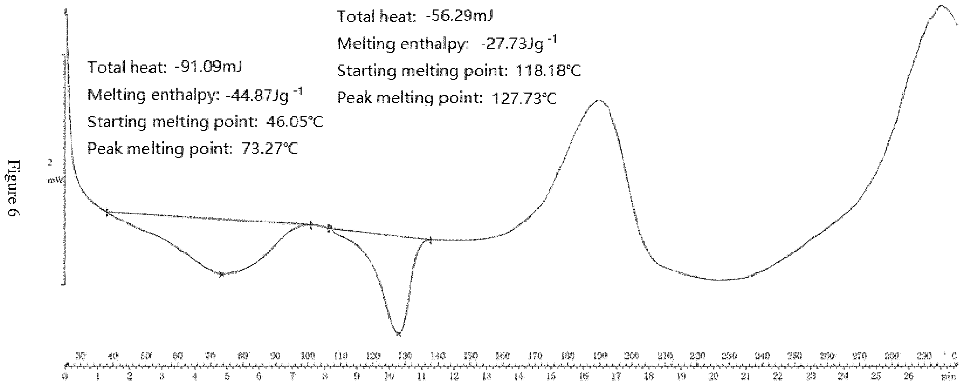

Approximately 20 mg of the compound represented by formula (II) was weighed and 4 mg of maleic acid was added thereto, then 200 µL of acetone was added and stirred for dissolution at room temperature, the stirring was continued, and solids were precipitated, the mixture was centrifuged, and the solid sample was dried in vacuum to obtain the III crystal form of the compound represented by formula (I). Its X-ray diffraction pattern spectrum is shown in Figure 5, the DSC pattern spectrum is shown in Figure 6, and the characteristic peak positions are shown in the table below:

**Table 3: characteristic peak positions of the III crystal form**

| Number of the peaks | 2θ[°] | d[Å] | I[%] |
|---|---|---|---|
| Peak 1 | 4.864 | 18.15286 | 12.6 |
| Peak 2 | 5.516 | 16.00949 | 8.7 |
| Peak 3 | 6.291 | 14.03761 | 43.9 |
| Peak 4 | 6.547 | 13.48974 | 100.0 |
| Peak 5 | 8.068 | 10.94991 | 8.0 |
| Peak 6 | 8.561 | 10.31997 | 29.6 |
| Peak 7 | 9.908 | 8.92036 | 14.9 |
| Peak 8 | 10.401 | 8.49865 | 30.3 |
| Peak 9 | 11.603 | 7.62067 | 36.5 |
| Peak 10 | 13.267 | 6.66808 | 14.9 |
| Peak 11 | 13.819 | 6.40329 | 19.5 |
| Peak 12 | 14.725 | 6.01129 | 14.1 |
| Peak 13 | 16.270 | 5.44375 | 12.4 |
| Peak 14 | 17.381 | 5.09798 | 23.6 |
| Peak 15 | 18.398 | 4.81836 | 4.8 |
| Peak 16 | 19.326 | 4.58904 | 31.9 |
| Peak 17 | 20.125 | 4.40876 | 8.2 |
| Peak 18 | 21.040 | 4.21905 | 18.2 |
| Peak 19 | 21.498 | 4.13015 | 10.5 |
| Peak 20 | 22.250 | 3.99231 | 4.6 |
| Peak 21 | 23.741 | 3.74469 | 31.3 |
| Peak 22 | 24.426 | 3.64129 | 10.8 |
| Peak 23 | 25.795 | 3.45105 | 19.1 |
| Peak 24 | 26.765 | 3.32819 | 5.5 |
| Peak 25 | 28.530 | 3.12612 | 11.2 |
| Peak 26 | 31.815 | 2.81042 | 11.7 |

### Embodiment 8

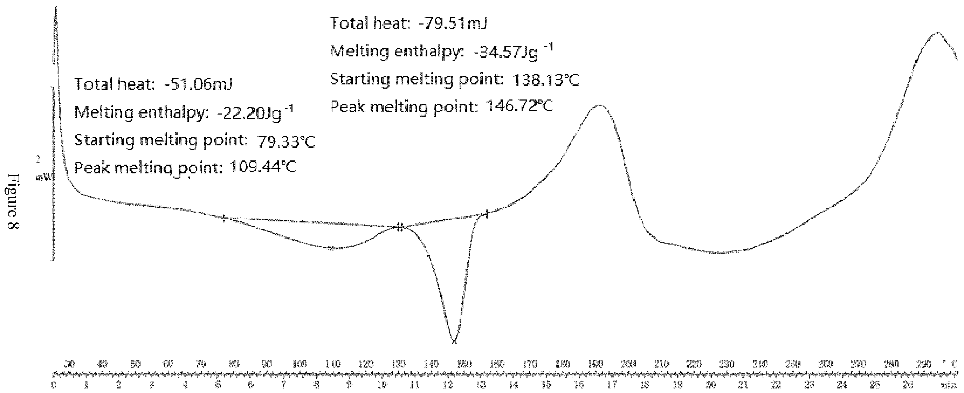

Approximately 20 mg of the compound represented by formula (II) was weighed and 4 mg of maleic acid was added thereto, then 200 µL of ethanol was added and stirred at room temperature for dissolution, the stirring was continued and solids were precipitated, the mixture was slurried at room temperature, then centrifuged, the solid sample was dried in vacuum to obtain the IV crystal form of the compound represented by formula (I). Its X-ray diffraction pattern spectrum is shown in Figure 7, the DSC pattern spectrum is shown in Figure 8, and the characteristic peak positions are shown in the table below:

**Table 4: characteristic peak positions of the IV crystal form**

| Number of the peaks | 2θ[°] | d[Å] | I[%] |
|---|---|---|---|
| Peak 1 | 5.638 | 15.66303 | 15.5 |
| Peak 2 | 8.268 | 10.68476 | 2.8 |
| Peak 3 | 8.772 | 10.07253 | 5.0 |
| Peak 4 | 9.417 | 9.38383 | 17.1 |
| Peak 5 | 11.054 | 7.99762 | 50.0 |
| Peak 6 | 12.386 | 7.14073 | 26.7 |
| Peak 7 | 13.739 | 6.44002 | 12.0 |
| Peak 8 | 15.218 | 5.81732 | 22.3 |
| Peak 9 | 15.639 | 5.66189 | 42.8 |
| Peak 10 | 16.312 | 5.42975 | 4.6 |
| Peak 11 | 17.074 | 5.18907 | 31.0 |
| Peak 12 | 18.369 | 4.82595 | 40.1 |
| Peak 13 | 19.152 | 4.63046 | 12.9 |
| Peak 14 | 20.439 | 4.34161 | 9.2 |
| Peak 15 | 21.907 | 4.05396 | 6.9 |
| Peak 16 | 22.307 | 3.98207 | 12.9 |
| Peak 17 | 22.779 | 3.90071 | 26.5 |
| Peak 18 | 23.414 | 3.79636 | 100.0 |
| Peak 19 | 24.146 | 3.68285 | 16.5 |
| Peak 20 | 24.837 | 3.58196 | 3.2 |
| Peak 21 | 25.384 | 3.50594 | 36.5 |
| Peak 22 | 25.852 | 3.44352 | 11.9 |
| Peak 23 | 26.426 | 3.37005 | 45.6 |
| Peak 24 | 26.774 | 3.32706 | 17.7 |
| Peak 25 | 28.685 | 3.10956 | 37.1 |
| Peak 26 | 29.782 | 2.99752 | 6.5 |
| Peak 27 | 31.620 | 2.82733 | 4.0 |
| Peak 28 | 32.482 | 2.75427 | 7.5 |

### Embodiment 9

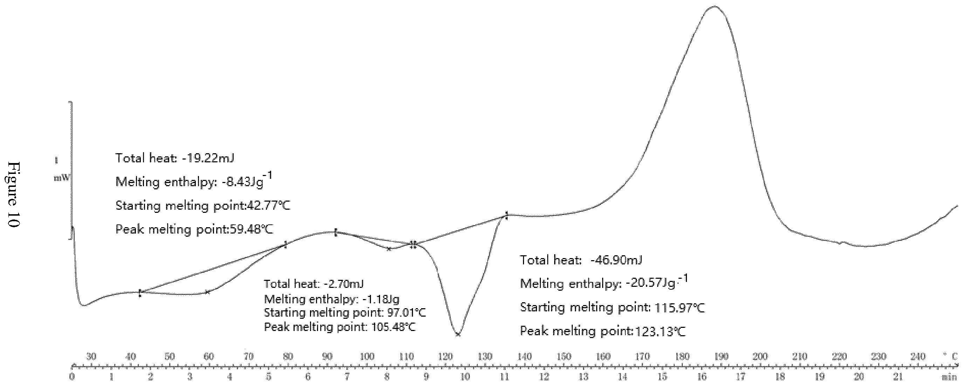

Approximately 20 mg of the compound represented by formula (II) was weighted and 4 mg of maleic acid was added thereto, then 400 µL of 1,4-dioxane was added and the temperature was raised to 50°C for reaction, after the completion of the reaction, the mixture was centrifuged and the solid sample was dried in vacuum to obtain the V crystal form of the compound represented by formula (I). Its X-ray diffraction pattern spectrum is shown in Figure 9, the DSC pattern spectrum is shown in Figure 10, the characteristic peak positions are shown in the table below.

**Table 5: characteristic peak positions of the V crystal form**

| Number of the peaks | 2θ[°] | d[Å] | I[%] |
|---|---|---|---|
| Peak 1 | 5.469 | 16.14750 | 36.4 |
| Peak 2 | 5.477 | 16.12122 | 37.8 |
| Peak 3 | 6.512 | 13.56204 | 100.0 |
| Peak 4 | 10.376 | 8.51906 | 15.8 |
| Peak 5 | 11.593 | 7.62717 | 32.2 |
| Peak 6 | 13.220 | 6.69166 | 5.0 |
| Peak 7 | 14.708 | 6.01819 | 6.1 |
| Peak 8 | 15.600 | 5.67590 | 7.8 |
| Peak 9 | 16.492 | 5.37076 | 6.0 |
| Peak 10 | 18.241 | 4.85972 | 20.0 |
| Peak 11 | 19.386 | 4.57517 | 25.7 |
| Peak 12 | 21.028 | 4.22145 | 26.2 |
| Peak 13 | 22.286 | 3.98581 | 21.6 |
| Peak 14 | 22.747 | 3.90608 | 10.1 |
| Peak 15 | 23.758 | 3.74218 | 15.8 |
| Peak 16 | 24.693 | 3.60252 | 4.0 |
| Peak 17 | 25.509 | 3.48913 | 13.2 |
| Peak 18 | 25.926 | 3.43386 | 21.8 |
| Peak 19 | 26.563 | 3.35304 | 3.2 |
| Peak 20 | 27.837 | 3.20233 | 8.2 |
| Peak 21 | 29.792 | 2.99653 | 3.8 |
| Peak 22 | 30.727 | 2.90746 | 4.3 |
| Peak 23 | 32.086 | 2.78729 | 5.8 |

### Embodiment 10

The stability of the I crystal form of the compound represented by formula (I) was investigated. The purity of the crystal form was detected by Agilent1200 DAD high performance liquid chromatography system, and Waters symmetry C18, (250*4.6mm, 5µm) was used as the detection column, mobile phase: sodium dihydrogen phosphate /ACN/H₂O, detection wavelength: 261nm.

**Table 6: Experimental results of the influencing factors for the I crystal form and amorphous from of the compound represented by formula (I)**

| sample/ purity % | Time (day) | illumination | 40°C | RH75% | RH90% |
|---|---|---|---|---|---|
| I crystal form | 0 | 99.56 | 99.56 | 99.56 | 99.56 |
| | 5 | 99.35 | 99.55 | 99.53 | 99.53 |
| | 10 | 99.29 | 99.51 | 99.53 | 99.54 |
| | 30 | 99.00 | 99.43 | 99.52 | 99.51 |
| amorphous | 0 | 99.31 | 99.31 | 99.31 | 99.31 |
| | 5 | 98.04 | 99.21 | 99.35 | 95.76 |
| | 10 | 96.65 | 99.13 | 99.33 | 94.05 |
| | 30 | 93.48 | 98.57 | 99.09 | 85.09 |

It can be seen from the table that after long-term storage, I crystal form has good physical and chemical stability; while the amorphous form has poor stability under illumination, high temperature and high humidity conditions.

### Embodiment 11

The stability of the I and IV crystal form of the compound represented by formula (I) was investigated. The purity of the crystal forms was detected by Thermo Ultimate3000DAD high-performance liquid chromatography system, and Waters symmetry C18, (250*4.6mm, 5µm) was used as the detection chromatographic column, mobile phase: sodium dihydrogen phosphate/ACN/H₂O, detection wavelength: 261nm.

**Table 7: Experimental results of the influencing factors for the I and IV crystal form of the compound represented by formula (I)**

| sample/ purity % | Time (day) | illumination | 40°C | 60°C | RH75% | RH90% |
|---|---|---|---|---|---|---|
| I crystal form | 0 | 99.83 | 99.83 | 99.83 | 99.83 | 99.83 |
| | 5 | 99.68 | 99.78 | 99.64 | 99.81 | 99.81 |
| | 10 | 99.59 | 99.77 | 99.51 | 99.81 | 99.82 |
| | 30 | 99.29 | 99.69 | 99.24 | 99.68 | 99.82 |
| IV crystal form | 0 | 99.94 | 99.94 | 99.94 | 99.94 | 99.94 |
| | 5 | 99.92 | 99.92 | 99.83 | 99.93 | 99.93 |
| | 10 | 99.92 | 99.93 | 99.77 | 99.94 | 99.94 |
| | 30 | 99.85 | 99.85 | 99.50 | 99.94 | 99.93 |

Although the specific embodiments of the present disclosure have been described above, those skilled in the art should understand that these are only examples, various changes or modifications can be made to these embodiments without departing from the principle and essence of the present invention. Therefore, the protection scope of the present disclosure is defined by the appended claims.

## Claims

1. A I crystal form of a compound represented by formula (I), which has an X-ray powder diffraction pattern spectrum comprising characteristic peaks at 2θ angles of 6.57, 8.12, 9.76, 10.77, 14.98, 15.89, 20.97, 21.64, 22.06 and 22.61,

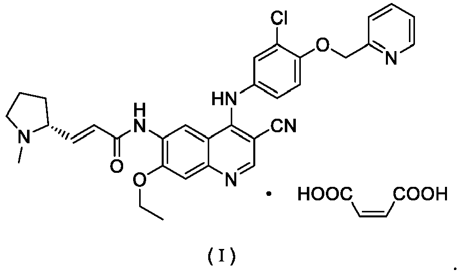

2. The I crystal form of the compound represented by formula (I) as defined in claim 1, which has an X-ray powder diffraction pattern spectrum comprising characteristic peaks at 2θ angles of 6.57, 8.12, 9.76, 10.77, 12.42, 13.11, 14.47, 14.98, 15.28, 15.89, 16.29, 16.49, 17.13, 17.46, 18.92, 19.56, 19.83, 20.29, 20.97, 21.64, 22.06, 22.61, 22.99 , 24.00, 24.60, 25.62, 26.46, 27.30, 27.99, 29.05, 30.19, 30.69, 31.90, 33.88 and 36.07.

3. The I crystal form of the compound represented by formula (I) as defined in claim 1, which has an X-ray powder diffraction pattern spectrum as shown in Figure 1.

4. A II crystal form of the compound represented by formula (I), which has an X-ray powder diffraction pattern spectrum comprising characteristic peaks at 2θ angles of 6.340, 9.030, 10.232, 11.503, 18.282, 19.399, 20.865 and 21.558,

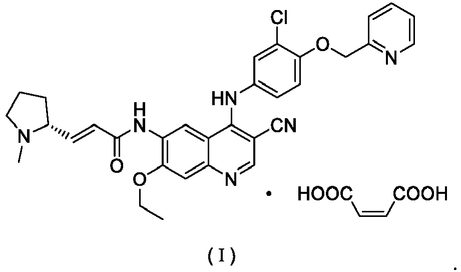

5. A III crystal form of the compound represented by formula (I), which has an X-ray powder diffraction pattern spectrum comprising characteristic peaks at 2θ angles of 6.291, 6.547, 8.561, 9.908, 10.401, 17.381, 19.326 and 23.741,

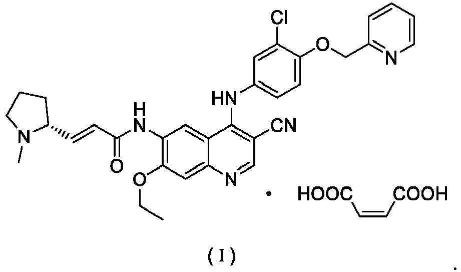

6. A IV crystal form of the compound represented by formula (I), which has an X-ray powder diffraction pattern spectrum comprising characteristic peaks at 2θ angles of 5.638, 9.417, 11.054, 12.386, 15.218, 15.639, 17.074 and 18.369,

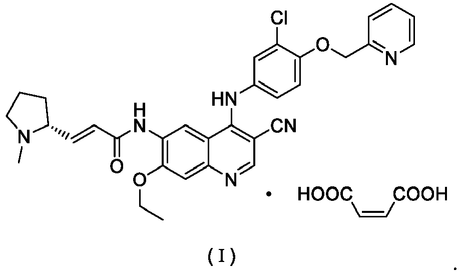

7. The IV crystal form of the compound represented by formula (I) as defined in claim 6, which has an X-ray powder diffraction pattern spectrum comprising characteristic peaks at 2θ angles of 5.638, 8.268, 8.772, 9.417, 11.054, 12.386, 13.739, 15.218, 15.639, 16.312, 17.074, 18.369, 19.152, 20.439, 21.907, 22.307, 22.779, 23.414, 24.146, 24.837, 25.384, 25.852, 26.426, 26.774, 28.685, 29.782, 31.620 and 32.482.

8. The IV crystal form of the compound represented by formula (I) as defined in claim 6, which has an X-ray powder diffraction pattern spectrum is as shown in Figure 7.

9. A V crystal form of the compound represented by formula (I), which has an X-ray powder diffraction pattern spectrum comprising characteristic peaks at 2θ angles of 5.469, 5.477, 6.512, 10.376, 11.593, 18.241, 19.386, 21.028 and 22.286,

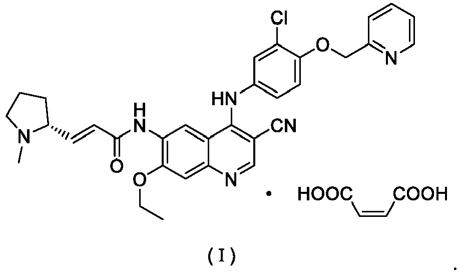

10. The crystal form of the compound represented by formula (I) as defined in any one of claims 1 to 9, wherein the error range of the 2θ angle is ±0.2.

11. A pharmaceutical composition prepared by mixing one or more of the I crystal form, II crystal form, III crystal form, IV crystal form and V crystal form of the compound represented by formula (I) as defined in any one of claims 1 to 10 with one or more pharmaceutically acceptable carriers, diluents or excipients.

12. A pharmaceutical composition comprising one or more of the I crystal form, II crystal form, III crystal form, IV crystal form and V crystal form of the compound represented by formula (I) as defined in any one of claims 1 to 10 and one or more pharmaceutically acceptable carriers, diluents or excipients.

13. A method for preparing the I crystal form of the compound represented by formula (I) as defined in any one of claims 1 to 3, wherein the method comprising: mixing and slurring the compound represented by formula (I) with a solvent and filtering the crystals obtained, the solvent is selected from one or more of water and tetrahydrofuran; or mixing the compound represented by formula (I) with a solvent to volatilize and crystallize, and the solvent is one or more selected from ethanol, isopropanol, n-propanol, acetone, acetonitrile, 2-butanone, dimethyl sulfoxide, nitromethane, propylene glycol methyl ether, isoamylol and acetophenone; or mixing the compound represented by formula (II) with maleic acid and a solvent to precipitate a solid and filtering the crystals obtained, the solvent is one or more selected from isopropanol, water, and dichloromethane, preferably a mixed solvent of isopropanol/water or dichloromethane,

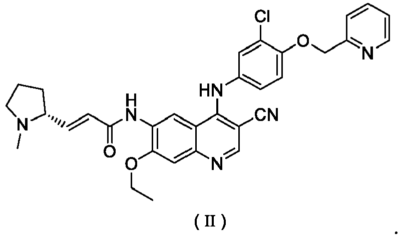

14. A method for preparing the II crystal form of the compound represented by formula (I) as defined in claim 4, wherein the method comprising: mixing and slurring a crystal form of the compound of formula (I) with tetrahydrofuran and filtering the crystals obtained, preferably, the crystal form of the compound represented by formula (I) is crystal form I.

15. A method for preparing the III crystal form of the compound represented by formula (I) as defined in claim 5, wherein the method comprising: mixing the compound represented by formula (II) with maleic acid and acetone to precipitate a solid, and filtering the crystals obtained,

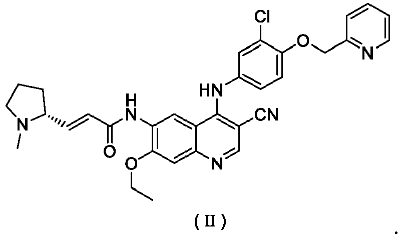

16. A method for preparing the IV crystal form of the compound represented by formula (I) as defined in any one of claims 6 to 8, the method comprising: mixing the compound represented by formula (II) with maleic acid and a solvent to precipitate a solid, and filtering the crystals obtained, the solvent can be one or more selected from n-propanol, isopropyl acetate, 2-butanone, isopropanol, and ethanol, preferably ethanol,

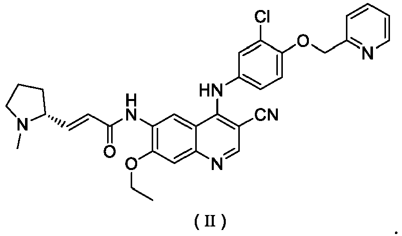

17. A method for preparing the V crystal form of the compound represented by formula (I) as defined in claim 9, the method comprising: mixing the compound represented by formula (II) with maleic acid and a solvent to precipitate a solid, filtering the crystals obtained, the solvent can be 1,4-dioxane and/or tetrahydrofuran,

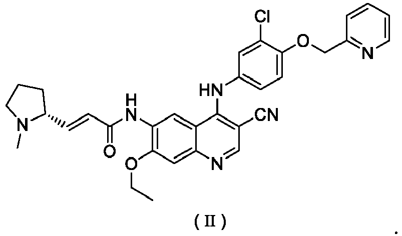

18. A method for preparing the pharmaceutical composition comprising the compound represented by formula (I) or the pharmaceutically acceptable salt thereof, wherein the method comprises mixing one or more of the I crystal form, the II crystal form, III crystal form, IV crystal form, and V crystal form of the compound represented by formula (I) as defined in any one of claims 1 to 10 with one or more pharmaceutically acceptable carriers, diluents or excipients.

19. The I crystal form, II crystal form, III crystal form, IV crystal form, V crystal form of the compound represented by formula (I) as defined in any one of claims 1-10, or the pharmaceutical composition as defined in any one of claims 11-12 for use in manufacturing a medicament for the treatment and/or prevention of a disease or a condition related to a protein kinase, wherein the protein kinase is selected from EGFR receptor tyrosine kinase or HER-2 receptor tyrosine kinase, the disease or condition is preferably cancer, and the cancer is preferably lung cancer, breast cancer, epidermal squamous cell carcinoma or gastric cancer.
